## Europäisches Patentamt

## European Patent Office

## Office européen des brevets

(19)

(11) Publication number: **0 085 402**
**B1**

# EUROPEAN PATENT SPECIFICATION

(12)

(45) Date of publication of patent specification: **04.02.87**

(21) Application number: **83100765.3**

(22) Date of filing: **27.01.83**

(51) Int. Cl.[4]: **G 01 N 33/531,**
**G 01 N 33/543, G 01 N 33/96,**
**C 12 Q 1/36**

(54) **Method of determination of human urine kallikrein and kit for the same.**

(30) Priority: **29.01.82 JP 13861/82**

(43) Date of publication of application:
**10.08.83 Bulletin 83/32**

(45) Publication of the grant of the patent:
**04.02.87 Bulletin 87/06**

(84) Designated Contracting States:
**BE DE FR GB LU NL SE**

(56) References cited:
**EP-A-0 031 993**

**J. Clin. Endocr. Met. 51-840-1980**
**J. Immunology 126-2361-1981**
**Principles of competitive Protein binding assay**
**303-315**

(73) Proprietor: **THE GREEN CROSS CORPORATION**
**15-1, Imabashi-1-chome Higashi-ku Osaka-shi**
**Osaka 541 (JP)**

(72) Inventor: **Moriya, Hiroshi**
**14, Nishiochiai-2-chome**
**Shinjuku-ku Tokyo (JP)**
Inventor: **Morichi, Shigehiro**
**3, Tsurukabuto-4-chome**
**Nada-ku Kobe (JP)**
Inventor: **Sako, Eiji**
**12-9, Nagaremachi-4-chome**
**Hirano-ku Osaka (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 085 402 B1

## Description

This invention relates to a kit for the quantitative determination of kallikrein in human urine by radioimmunoassay for the diagnosis of various diseases including hypertension.

Kallikrein is an enzyme which acts upon kininogen in plasma, and liberates kinin which is a physiologically active peptide.

The biological actions of kallikrein are developed through the liberated kinin. These actions include, for example, vasodilation, blood pressure lowering, smooth muscle contraction, capillary vessel permeability raising, catecholamine secretion raising, and prostaglandin biosynthesis acceleration.

It is known that kallikrein is excreted not only in human urine but also in the urine of many animals. The kallikrein in urine is produced by the biosynthesis in the kidney, and participates particularly in the control of renal blood flow and blood pressure.

Thus kallikrein plays an important role in maintaining the constancy of a living body, showing a broad range of physiological activities. Accordingly, the decrease of biosynthesis or secretion of kallikrein gives rise to serious diseases. It is known, for instance, that the amount of kallikrein excreted in the urine of a patient suffering from essential or nephrogenous hypertension is distinctly low compared with that of a normal person, and that the amount of kallikrein excreted in urine is large in case of primary aldosteronism or Bartter's syndrome and is small in case of renal insufficiency.

Thus, in treating various diseases including hypertension, it is highly useful for the diagnosis of the diseases to determine quantitatively the kallikrein excreted in urine.

Known methods of quantitative determination of kallikrein include a method in which the lowering of whole body blood pressure of a dog administered with the specimen intravenously is used as an index of the amount of kallikrein [Frey, E. K., Arch. Klin. Chir., *142*, 663 (1926)] and a method in which the hydrolytic activity of kallikrein on various synthetic substrates such as methyl ester of tosylarginine, ethyl ester or benzoylarginine, prolyl-phenylalanylarginine methylcoumarinamide, and D-valyl-leucyl-arginine para-nitroanilide is estimated. These methods are not only complicated in their procedure but poor in the specificity of the determination.

In recent years immunoassays, particularly radioimmunoassay, utilizing the specificity of antigen-antibody reaction have been developed, and it has been reported that kallikrein can be accurately determined by said assay.

As is apparent from these reports by the application of radioimmunoassay an accurate determination of trace amount of kallikrein excreted in urine has become possible, and the correlation between various diseases and the amounts of kallikrein excreted in urine has come to be investigated in detail.

In the methods disclosed in these reports, however, the reaction of kallikrein labelled with a radioactive substance and kallikrein in the specimen (urine) which act as antigens with the anti-kallikrein antibody acting as an antibody is carried out in liquid phase, so that the separation of the antigen labelled with a radioactive substance which has formed a complex (B-body) with the antibody from the labelled antigen remaining free (F-body) is troublesome. In the prior methods mentioned above B/F separation is carried out by the double antibody method (N.B. Oza et al., J. of Immunol. 126, 2361 (1981)) or polyethylene-glycol precipitation method (K. Shimmoto et al., J. Clin. Endoc. and Metab. 51, 840 (1980)). In these methods of separation, however, incubation or centrifugation is necessary for separation, so that the time required for measurement is lengthened and the operations are complicated.

Accordingly, it is an urgent problem to find a method in which the B/F separation can be done easily, and the determination of kallikrein in urine can be carried out rapidly and accurately with a large number of urine specimens. The object of this invention is to provide a kit for the quantitative determination of kallikrein in urine by which the above problem would be solved.

According to this invention, there is provided a kit for quantitative determination of human urine kallikrein which comprises

a) a vessel having an effective amount of anti-human urine kallikrein-antibody immobilized therein,

b) human urine kallikrein labelled with a radioactive substance,

c) standard human urine kallikrein, and

d) a buffer solution for the antigen-antibody reaction, characterized in that the anti-human urine kallikrein-antibody is immobilized in the vessel at about pH 5.5 and at an ion concentration of about 0.01M.

This invention also provides a process for the production of a vessel having an effective amount of anti-human urine kallikrein-antibody immobilized therein, comprising the step of immobilizing the anti-human urine kallikrein-antibody in the vessel at about pH 5.5 and at an ion concentration of about 0.01M.

The standard human urine kallikrein used in the kit of this invention is employed for the prapration of the standard calibration line mentioned later, and comprises usually a lyophilized product of urine kallikrein. Standard human urine kallikrein prepared by any method may be used so long as it has high purity. For instance, a lyophilized product of human urine kallikrein is favorably used which is obtained, for example, by a method in which fresh human urine is contacted with colloidal hydrated aluminium silicate and then subjected to affinity chromatography by use of insoluble trypsin inhibitor. Standard human urine kallikrein is used after dissolving it in a buffer solution in 4 to 5 steps of concentration ranging, for example, from 1 to 100 nm/ml for preparing the standard calibration line.

Preferred human urine kallikrein labelled with a radioactive substance is, for example, the one labelled with $^{125}$I or $^{131}$I, and suitably has a specific radioactivity of, in the case of $^{125}$I for instance, about $10^5$ to $10^7$ cpm per 1 μg of human urine kallikrein. The human urine kallikrein labelled with a radioactive substance can be prepared by labelling the kallikrein in a manner known *per se* such as the chloramine T method [Greenwood, F. C. et al., Biochem J., *89*, 114 (1963)], the lactoperoxidase method [Miyashi Y., et al., J. Clin. Endocrinol. Metabl., *34*, 23 (1972)] and the Bolten-Hunter's reagent method [Bolten, A. E., and Hunter, W. M., Biochem. J. *133*, 529 (1973)].

The vessel in which the anti-human urine kallikrein antibody is immobilized is preferably in the form of a test tube, and preferably has a diameter of about 5 to 15 mm and a length of about 50 to 100 mm. Suitable materials thereof include synthetic resins such as polystyrene, polypropylene or polyethylene and glass. Synthetic resins are preferred because of their easy handling and low price.

The anti-human urine kallikrein to be immobilized in the said vessel can be obtained by sensitizing an animal such as a rabbit, guinea pig or goat with human urine kallikrein after, for example, emulsifying it together with Freund's adjuvant. The purification of anti-human urine kallikrein antibody from an animal antiserum can be performed by a known method using such treatment as ammonium sulfate precipitation, ion-exchange chromatography or molecular sieving either alone or in combination.

As method for immobilizing the anti-human urine kallikrein antibody onto the vessel, there can be cited, the following method. The anti-human urine kallikrein antibody is diluted with a buffer solution having a pH of 5 to 10 about 100-, 400-, 1600-, 6400-, 25600-, 102400 and 409600-fold. As buffer solutions there may be used those from salts of acetic acid, phosphoric acid and Tris-hydrochloric acid, and particularly 0.01 M sodium acetate buffer solution (pH 5.5) is preferred. A suitable amount (e.g. 0.5 to 1.5 ml) of a diluted solution of said antibody is placed in the vessel onto which the antibody is to be immobilized and, after standing at 0 to 37°C for 1 to 24 hours, the diluted solution is removed by suction. The vessel is washed preferably 1 to 3 times by using a suitable amount (e.g. 1 to 2 ml) of a washing solution. As washing solution there may be used distilled water, physiological saline, or a solution which is formed by adding and dissolving thereto 0.01 to 0.1% of albumin or Tween®-20. After washing, the vessel is dried to give a vessel having antihuman urine kallikrein immobilized therein.

In the vessel containing the immobilized antibody a kallikrein labelled with a radioactive substance, for example the $^{125}$I-labelled kallikrein is placed in an amount corresponding to about 10,000 cpm., and the total volume is brought preferably to 1 ml by adding a buffer solution (pH 6 to 9). After 2 to 24 hours at 0 to 37°C the vessel is washed in a similar manner mentioned above and the radioactivity is measured. For the kit of the present invention it is preferable to use a vessel with immobilized antibody which is capable of binding about 40 to 60% of the employed radio labelled kallikrein.

The buffer solution used for the kit of this invention is suitable for antigen-antibody reaction, thus it has preferably a pH value of 6 to 9 and a salt concentration of 0.01 to 0.2 M. Specific examples thereof include 0.1 M phosphate-buffered physiological saline (pH 7).

The method of determination of human urine kallikrein using the kit of this invention is described in the followings.

Method of determination

A buffered solution of human urine kallikrein labelled with a radioactive substance and a sample fluid (urine specimen) are placed in a vessel having anti-human urine kallikrein antibody immobilized therein, and the whole is incubated at 0 to 37°C for 2 to 24 hours. Thus, a complex (B-body) is formed by the antigen-antibody reaction of the immobilized antibody with the human urine radio labelled kallikrein and the kallikrein in sample fluid. Since the anti-human urine kallikrein antibody is immobilized on the vessel in this invention, the separation of the free radio labelled kallikrein and the free kallikrein in the sample fluid (these correspond namely to F-body) can be performed by removing the liquid in the vessel by suction, decantation or other suitable means. Thus, the B/F separation can be performed very easily in this invention.

After the B/F separation, the radioactivity of the vessel is measured. Thus, the larger the amount of kallikrein in the sample fluid, the smaller the coupled amount of human urine radio labelled kallikrein and so the radioactivity.

Preparation of the standard calibration curve The standard calibration curve is prepared by repeating the above-mentioned determination procedure using standard human urine kallikrein solution of various concentrations in place of the above sample fluid. The amount of kallikrein in the sample fluid is determined by comparing the value of radioactivity of the sample fluid with those in the calibration line.

The standard calibration line is prepared as follows:

On a logit-log paper, for example, is indicated as ordinate the value of $B \times 100/B_0$ ($B_0$ being the radioactivity measured when the largest amount of human urine kallikrein labelled with a radioactive substance alone is coupled with the anti-human urine kallikrein immobilized on the vessel; B being the radioactivity measured when standard human urine kallikrein at each concentration and human kallikrein labelled with a radioactive substance are coupled) and as abscissa the amount of human urine kallikrein (ng/vessel). The standard calibration line is prepared by connecting the plots obtained with standard human urine kallikrein at various concentrations.

The determination of the amount of human urine kallikrein (ng/vessel) contained in the sample fluid can then be determined by measuring the values of $B \times 100/B_0$ of each sample fluid and comparing these with those in said standard calibration line.

Example 1
Preparation of a vessel (test tube) having anti-human urine kallikrein antibody immobilized therein.

An anti-human urine kallikrein antibody was obtained from a rabbit antiserum after purification by ammonium sulfate precipitation method and DEAE-cellulose chromatography. It was then diluted 6400-fold by using 0.01 M sodium acetate solution (pH 5.5). One ml of this diluted solution was placed in a polystyrene test tube (1.0×7.5 cm) and allowed to stand at 37°C for 4 hours. After removing the liquid in the test tube by suction, the tubes was washed twice with 1.5 ml of physiological saline containing 0.05% of bovine serum albumin and further once with physiological saline containing 0.01% $NaN_3$, and then dried at 25°C with aeration.

Example 2
Determination of kallikrein in urine by use of the kit of this invention.

In a test tube having anti-human urine kallikrein immobilized therein were placed 0.8 ml of phosphate-buffered physiological saline, 0.1 ml of $^{125}$I-labelled kallikrein solution (containing an amount corresponding to about 10,000 cpm.) and 0.1 ml of urine specimen from the subject shown in the table. After stirring, the whole was allowed to stand at 37°C for 4 hours. After removing the liquid in the test tube by suction, the tube was washed twice using 1.5 ml of distilled water, and then the radioactivity was measured by use of a gamma-counter. In the mean time, the standard calibration line was prepared with reaction systems in each of which, in place of the urine specimen in the above-mentioned reaction solution, 0.1 ml of the standard kallikrein solution of various concentrations (containing 1, 4, 16, 64 and 128 ng as kallikrein respectively) was used. The amount of human urine kallikrein contained in the urine specimen was determined by comparing the radioactivity of the test tube in which the urine specimen had been used for reaction with those in the calibration line.

The results were shown in Table 1.

TABLE 1

| Subjects under examination | Kallikrein in urine |
| --- | --- |
| Normal adults with normal blood pressure (n=27) | 100.1±21.3 ng/ml |
| Patients with essential hypertension (n=13) | 68.2±18.7 ng/ml |
| Patients with Bartter's syndrome (n=2) | 788.3±51.1 ng/ml |

Note: n is the number of subjects.

**Claims**

1. A kit for quantitative determination of human urine kallikrein which comprises
   a) a vessel having an effective amount of anti-human urine kallikrein-antibody immobilized therein,
   b) human urine kallikrein labelled with a radioactive substance,
   c) standard human urine kallikrein, and
   d) a buffer solution for the antigen-antibody reaction,
   characterized in that the anti-human urine kallikrein-antibody is immobilized in the vessel at about pH 5.5 and at an ion concentration of about 0.01M.

2. Process for the production of a vessel having an effective amount of anti-human urine kallikrein-antibody immobilized therein, comprising the step of immobilizing the anti-human urine kallikrein-antibody in the vessel at about pH 5.5 and at an ion concentration of about 0.01M.

**Patentansprüche**

1. Ein Besteck zur quantitativen Bestimmung von menschlichem Urin-Kallikrein, das
   a) ein Gefäß mit einer wirksamen Menge immobilisiertem Antikörper gegen menschliches Urin-Kallikrein,
   b) mit einer radioaktiven Substanz markiertes menschliches Urin-Kallikrein,
   c) menschliches Urin-Kallikrein als Standard, und
   d) eine Pufferlösung für die Antigen-Antikörper-Reaktion enthält, dadurch gekennzeichnet, daß der Antikörper gegen menschliches Urin-Kallikrein in dem Gefäß bei etwa 5,5 und eine Ionenkonzentration von etwa 0,01 M immobilisiert ist.

2. Verfahren zur Herstellung eines Gefäßes mit einer wirksamen Menge darin immobilisiertem Antikörper gegen menschliches Urin-Kallikrein, bei dem man die Immobilisierung des Antikörpers gegen menschliches Urin-Kallikrein im Gefäß bei etwa pH 5,5 und einer Ionenkonzentration von etwa 0,01 M durchführt.

**Revendications**

1. Trousse pour la détermination quantitative de la kallikréine d'urine humaine, qui comprend:

a) un récipient dans lequel est immobilisée une quantité efficace d'anti-corps anti-kallikréine d'urine humaine,

b) de la kallikréine d'urine humaine marquée avec une substance radioactive,

c) de la kallikréine d'urine humaine étalon. et

d) une solution tampon pour la réaction antigène-anticorps,

caractérisé en ce que l'anti-corps anti-kallikréine d'urine humaine est immobilisé dans le récipient à un pH d'environ 5,5 et à une concentration en ions d'environ 0,01 M.

2. Procédé pour la production d'un récipient dans lequel est immobilisée une quantité efficace d'un anti-corps anti-kallikréine d'urine humaine, comprenant l'étape consistant à immobiliser l'anti-corps anti-kallikréine d'urine humaine dans le récipient à un pH d'environ 5,5 et à une concentration en ions d'environ 0,01 M.